# EUROPEAN PATENT SPECIFICATION

(11) **EP 2 235 158 B1**
(45) Date of publication and mention of the grant of the patent: **25.12.2013**
(21) Application number: 08778856.8
(22) Date of filing: 18.07.2008
(51) Int. Cl.: C12N 1/20, A23L 1/30, A61K 35/74

(54) **METHOD OF PREPARING TRIPLE-COATING LACTIC ACID BACTERIA AND NANO PARTICLE COATING METHOD, TRIPLE-COATING LACTIC ACID BACTERIA PREPARED THEREBY AND ARTICLE COMPRISING THE SAME**
VERFAHREN ZUR HERSTELLUNG VON DREIFACH BESCHICHTETEN MILCHSÄUREBAKTERIEN UND BESCHICHTUNGSVERFAHREN UNTER VERWENDUNG VON NANOPARTIKELN, DAMIT HERGESTELLTE DREIFACH BESCHICHTETE MILCHSÄUREBAKTERIEN UND DIESE ENTHALTENDER ARTIKEL
MÉTHODE DE PRÉPARATION DE BACTÉRIES PRODUCTRICES D'ACIDE LACTIQUE À TRIPLE REVÊTEMENT, MÉTHODE DE REVÊTEMENT DE NANOPARTICULES, BACTÉRIES PRODUCTRICES D'ACIDE LACTIQUE À TRIPLE REVÊTEMENT AINSI PRÉPARÉES, ET ARTICLE LES COMPRENANT

(30) Priority: 25.01.2008 KR 20080008267
(43) Date of publication of application: 06.10.2010
(73) Proprietor: Cell Biotech Co., Ltd., Gyeonggi-do 415-872 (KR)
(72) Inventor: CHUNG, Myung Jun, Seoul 137-070 (KR); KIM, Dong Myung, Seoul 158-070 (KR); KIM, Jin Eung, Tongjin-eup, Gimpo-si Gyeonggi-do 415-770 (KR); LEE, Gwa Soo, Seongnam-si Gyeonggi-do 462-122 (KR)
(74) Representative: Bockhorni & Kollegen
(86) International application number: PCT/KR2008/004199
(87) International publication number: WO 2009/093785

(56) References cited:
- WO-A1-2005/030229
- JP-A- 5 186 337
- KR-A- 20020 069 863
- KR-A- 20070 104 140
- KR-B1- 100 782 984
- REIS CATARINA P ET AL: "Review and current status of emulsion/dispersion technology using an internal gelation process for the design of alginate particles", JOURNAL OF MICROENCAPSULATION, vol. 23, no. 3, May 2006 (2006-05), pages 245-257, XP002631972, ISSN: 0265-2048
- ANNAN N T ET AL: 'Encapsulation in alginate-coated gelatin microspheres improves survival of the probiotic Bifidobacterium adolescentis 15703T during exposure to simulated gastro-intestinal conditions' FOOD RESEARCH INTERNATIONAL vol. 41, no. 2, 17 November 2007, pages 184 - 193, XP022475730 ISSN: 0963-9969
- HORNIG STEPHANIE ET AL: "Nanoscale structures of dextran esters", CARBOHYDRATE POLYMERS, vol. 68, no. 2, March 2007 (2007-03), pages 280-286, XP005892443, ISSN: 0144-8617
- CRITTENDEN ROSS ET AL: "Synbiotic microcapsules that enhance microbial viability during nonrefrigerated storage and gastrointestinal transit.", APPLIED AND ENVIRONMENTAL MICROBIOLOGY MAR 2006 LNKD- PUBMED:16517688, vol. 72, no. 3, March 2006 (2006-03), pages 2280-2282, XP002631975, ISSN: 0099-2240

## Description

### [Technical Field]

This application claims priority to Korean Patent Application No.10-2008-0008267, filed on January 25, 2008.

The present invention relates to a method for preparing triple-coated lactic acid bacteria, triple-coated lactic acid bacteria prepared thereby, and an article comprising the same. More particularly, the present invention relates to a method for preparing lactic acid bacteria triple-coated with protein, polysaccharide and nanoparticles, triple-coated lactic acid bacteria prepared thereby, and an article comprising the same.

### [Background Art]

The lactic acid bacteria live in the intestines of mammals and prevent abnormal fermentation by saprophytes. Thus, they are utilized as medicine for intestinal disorders. For example, *L. bulgaricus* is the lactic acid bacteria that have been known from long ago. It is used for the production of yogurt and also as starter when producing cheese or cultured butter. And, the aerobic lactic acid bacteria *L. acidophilus,* which is found in the intestines of all mammals including human and other animals, is used for the production of butter or milk or for the treatment of autointoxication. Meanwhile, *L. lactis* produces D- and L-lactic acids. It is used for the production of butter or cheese, and is the most important lactic acid bacteria for dairying.

The lactic acid bacteria settle in the intestine and provide various physiological activities, including activation of intestinal movement, inhibition of harmful bacteria, promotion of synthesis of vitamins and immunostimulants, and the like. When orally taken, lactic acid bacteria tend to be killed by gastric acid or bile acid and do not exert their physiological activities.

In order to solve this problem, techniques for coating lactic acid bacteria have been developed. Conventional techniques for coating lactic acid bacteria include enteric-coated lactic acid bacteria using capsules and microencapsulated lactic acid bacteria using gelatin, sugar, gum, and the like.

Specifically, the conventional techniques for coating lactic acid bacteria are characterized by a separate coating process following the collection of lactic acid bacteria. More specifically, the conventional lactic acid bacteria coating process is performed by adding an aqueous coating composition capable of forming ultrafine spherical beads to lactic acid bacteria powder, and carrying out stirring, mixing and lyophilization.

Because the conventional techniques for coating lactic acid bacteria comprise collecting cultured lactic acid bacteria and making them into powder after drying, followed by mixing with a coating composition and stirring, they are associated with the following problems. The use of expensive coating agent and addition of the coating process result in increased production cost. Further, aseptic manipulation is difficult because other bacteria may be included. Besides, use of cryoprotectant and stabilizer during lyophilization following the coating for ensuring superior viability and stability may result in interactions between materials or redundancy of processes.

In order to solve these problem, double-coated lactic acid bacteria with improved bacterial stability and processing stability through prevention of direct interaction with air and moisture and through enhanced heat resistance, acid resistance and bile resistance have been developed [Korean Patent Application No. 2001-10397]. Specifically, this preparation method is characterized by preparing single-coated lactic acid bacteria using inexpensive protein, and then coating the bacteria again using polysaccharide such as xanthan gum, cellulose or levan.

However, even with the aforesaid improved techniques for coating lactic acid bacteria, it is not easy to completely coat the surface of lactic acid bacteria. Hence, the lactic acid bacteria prepared by the methods do not have superior heat resistance, acid resistance and bile resistance.

Nanotechnology refers to a field of applied science and technology whose theme is to find out peculiar properties and phenomenons of nano-sized materials (nanomaterials), and produce useful materials or systems by aligning and combining the nanomaterials. The methods for preparing nanomaterials that have been developed thus far are classified into a dry process utilizing combustion, explosion or shock wave; a sol-gel process; and a wet process utilizing supercritical fluid, chemical reaction, solvent dispersion, precipitation, evaporation, organic solvent, etc. Recently, the nanotechnology is applied in the food industry in order to make the foodstuff which was hard to be uptaken easy to take in, not in part but as a whole. As a result, the foodstuff which had to be processed chemically or thermally can be uptaken as it is. Further, through encapsulation of nanoparticles, it will be possible to prevent denaturation of nutrients and control their actions at the target area in the body. However, the application of the nanotechnology for coating lactic acid bacteria has never been reported as yet.

### [Disclosure]

### [Technical Problem]

The inventors of the present invention have developed a method for preparing triple-coated lactic acid bacteria with superior heat resistance, acid resistance and bile resistance by coating coated lactic acid bacteria with protein, polysaccharide and nanoparticles using the nanotechnology.

Accordingly, an object of the present invention is to provide a method for preparing triple-coated lactic acid bacteria using protein, polysaccharide and nanoparticles, triple-coated lactic acid bacteria prepared thereby, and an article comprising the same.

### [Technical Solution]

In an aspect, the present invention provides a method for preparing triple-coated lactic acid bacteria comprising the steps of:
(a) treating an aqueous protein solution with a protease;
(b) adding glucose, yeast extract, meat extract, an ionic component and lactic acid bacteria to the aqueous protein solution enzyme-treated in the step (a), and fermenting to prepare single-coated lactic acid bacteria;
(c) mixing the single-coated lactic acid bacteria prepared in the step (b) with an aqueous polysaccharide solution to prepare double-coated lactic acid bacteria; and
(d) coating the double-coated lactic acid bacteria prepared in the step (c) with nanoparticles to prepare triple-coated lactic acid bacteria, wherein the nanoparticles of the step (d) are solid lipid nanoparticles.

In another aspect, the present invention provides triple-coated lactic acid bacteria prepared by the preparation method.

In another aspect, the present invention provides an article comprising the triple-coated lactic acid bacteria.

### [Advantageous Effects]

The method for preparing triple-coated lactic acid bacteria according to the present invention provides lactic acid bacteria having better acid resistance, bile resistance and stability in accelerated test than conventional double-coated lactic acid bacteria by coating lactic acid bacteria with nanoparticles. Accordingly, the lactic acid bacteria prepared in accordance with the present invention are not destroyed by gastric acid or bile acid after intake but maintain inherent physiological activities. Therefore, they can be effectively utilized in various products including fermented milk, processed milk, fermented soybean products, fermented kimchi products, functional drinks, functional foods, conventional foods, cosmetics, and the like.

### [Brief Description of Drawings]

The above and other objects, features and other advantages of the present invention will be more clearly understood from the following description taken in conjunction with the accompanying drawings, in which:
Fig. 1 shows an image of triple-coated lactic acid bacteria coated with nanoparticles in accordance with the present invention; and
Fig. 2 shows an image of conventional double-coated lactic acid bacteria.

### [Best Mode for Carrying Out the Invention]

Hereinafter, the present invention is described in detail.

The method for preparing triple-coated lactic acid bacteria according to the present invention comprises the steps of:
(a) treating an aqueous protein solution with a protease;
(b) adding glucose, yeast extract, meat extract, an ionic component and lactic acid bacteria to the aqueous protein solution enzyme-treated in the step (a), and fermenting to prepare single-coated lactic acid bacteria;
(c) mixing the single-coated lactic acid bacteria prepared in the step (b) with an aqueous polysaccharide solution to prepare double-coated lactic acid bacteria; and
(d) coating the double-coated lactic acid bacteria prepared in the step (c) with nanoparticles to prepare triple-coated lactic acid bacteria, wherein the nanoparticles of the step (d) are solid lipid nanoparticles.

In the step (a), the aqueous protein solution preferably may be, although not limited thereto, 1-10% aqueous solution prepared from soy protein isolate or skim milk powder. And, the protease preferably may be, although not limited thereto, selected from the group consisting of pepsin, trypsin, chymotrypsin and elastase.

In an embodiment of the present invention, soy protein isolate and skim milk powder are suspended in water to prepare an aqueous protein solution, and then a protease (Protease-N, Amano) is added to the prepared aqueous solution (see Example 1-1).

The glucose, yeast extract, meat extract and ionic component added to the enzyme-treated aqueous protein solution of the step (a) in the step (b) are essential in the survival and growth of lactic acid bacteria. Preferably, although not limited thereto, 1-5 weight % of glucose, 0.1-1.5 weight % of yeast extract, 0.1-1.5 weight % of meat extract, and 0.01-0.1 weight % of ionic component may be added, based on the weight of the aqueous protein solution enzyme-treated in the step (a). Further, the ionic component preferably may be, although not limited thereto, at least one selected from the group consisting of ammonium citrate, sodium acetate, dipotassium phosphate, magnesium sulfate, manganese sulfate and sodium chloride. And, the lactic acid bacteria preferably may be, although not limited thereto, at least one selected from the group consisting of genus *Streptococcus,* genus *Lactococcus,* genus *Enterococcus,* genus *Lactobacillus,* genus *Pediococcus,* genus *Leuconostoc,* genus *Weissella* and genus *Bifidobacterium,* and the lactic acid bacteria may be preferably concentrated to a concentration of 10¹⁰-10¹¹ CFU/mL. In the step (b), the lactic acid bacteria are single-coated by protein, and are given heat resistance, acid resistance and bile resistance. However, only with such single coating, the surface of the lactic acid bacteria is not completely coated. A sterilization process may be added prior to cultivation of lactic acid bacteria, and a process of isolating and enriching the fermented solution of lactic acid bacteria may be added to induce deposition of remaining protein components along with the bacteria, which results in effective coating.

In an embodiment of the present invention, glucose, meat extract, yeast extract, and ionic component comprising dipotassium phosphate, ammonium citrate, sodium acetate, magnesium sulfate and manganese sulfate are added to the aqueous protein solution enzyme-treated in the step (a), and, after sterilization, the lactic acid bacteria *Lactobacillus acidophilus* (CBT-LH) are added. Then, single-coated lactic acid bacteria are prepared by isolation and enriching using a continuous centrifuge (see Example 1-1).

In the step (c), the single-coated lactic acid bacteria of the step (b) may be mixed with an aqueous polysaccharide solution to prepare lactic acid bacteria double-coated with polysaccharide. The aqueous polysaccharide solution preferably may be, although not limited thereto, an aqueous polysaccharide solution comprising 1-10% of polysaccharide based on the weight of the single-coated lactic acid bacteria of the step (b). And, the polysaccharide preferably may be, although not limited thereto, at least one polysaccharide selected from the group consisting of xanthan gum, cellulose and levan. In the step (c), the lactic acid bacteria are double-coated with polysaccharide. The strong adhesion property provided by the polysaccharide induces binding among the bacteria and, thus, results in bacterial clusters having a highly compact structure. Especially, in an aqueous solution, the polysaccharide has a very low solubility in acidic conditions and is easily dissolved and dissociated at neutral or higher pH. Hence, stability of the lactic acid bacteria under acidic conditions and settlement thereof of in the intestine can be improved significantly.

Further, in case the double-coated lactic acid bacteria prepared in the step (c) are desired to be lyophilized, a step of adding an aqueous cryoprotectant solution to the double-coated lactic acid bacteria of the step (c) and carrying out lyophilization according to a common method may be added. The aqueous cryoprotectant solution preferably may be, although not limited thereto, an aqueous solution comprising 1-10% of a cryoprotectant based on the weight of the single-coated lactic acid bacteria of the step (b). The cryoprotectant preferably may be, although not limited thereto, at least one selected from the group consisting of trehalose, maltodextrin, mannitol and skim milk powder.

In an embodiment of the present invention, the single-coated lactic acid bacteria prepared in the step (b) are mixed with an aqueous cryoprotectant solution comprising trehalose, mannitol and maltodextrin, and an aqueous polysaccharide solution comprising xanthan gum, cellulose and levan, and then lyophilization is carried out (see Example 1-1).

In the step (d), the nanoparticles are solid lipid nanoparticles (SLN). The SLN preferably may be at least one selected from the group consisting of lecithin, cholesterol, stearic acid, calcium stearate, and a combination thereof. Most preferably, the SLN may be stearic acid or soy lecithin. The SLN preferably may, although not limited thereto, have a particle size ranging from 100 to 200 nm.

Further, a step of carrying out filtration, microfiltration, centrifugation, ultracentrifugation, precipitation, decantation or a combination thereof according to a common nanoparticle preparation method may be added in order to partially separate the nanoparticles. Further, a step of destabilizing the suspension may be added. The destabilization process may be carried prior to the filtration, microfiltration, centrifuge, ultracentrifugation or precipitation process. Specifically, the destabilization step may comprise adding a destabilizing liquid to the suspension. The destabilizing liquid may be polar and can be mixed with a nonpolar liquid. The destabilizing liquid may be mixed with water. For example, the destabilizing liquid may be acetone, ethanol, methanol or some other liquid. The destabilization step may comprise, for example, changing temperate of the suspension to the temperature at which the suspension becomes unstable. Depending on situations, the temperature change can be accomplished by heating or cooling.

Further, a step of washing the nanoparticles may be included. The washing step may comprise contacting the nanoparticles with a washing liquid (aqueous or organic) and separating the nanoparticles from the washing liquid. For example, the washing step may comprise, in part or as a whole, suspending the nanoparticles in a washing liquid, agitating randomly collected washing liquid and nanoparticles, and separating the nanoparticles from the washing liquid, for example, using the separation method mentioned above. Alternatively, the washing step may comprise, in part or as a whole, passing the washing liquid through the nanoparticles which may be held, for example, in a filter. The washing may be carried out by phase separation using a decantation funnel. The aqueous washing liquid may be water or an aqueous liquid, for example, a salt solution. The organic washing liquid may be a solvent, which may be a polar or nonpolar solvent, for example, methanol, ethanol, isopropanol, acetone, dichloromethane, chloroform, ethyl acetate, toluene or some other solvents. A combination thereof may also be used. The washing step may comprise heating or cooling the suspension at about 10-70 °C, or at about 10-50, 10-30, 10-20, 20-70, 50-70, 20-50 or 30-50 °C, or heating or cooling the suspension at about 10, 15, 20, 25, 30, 35, 40, 45, 50, 55, 60, 65 or 70°C. The heating or cooling temperature may be the temperature at which the status of the system is changed so that the formation of a stable phase by the system is prevented. Further, a solvent mixture may be used to change the status of the system and, thereby, to prevent it from forming a stable single phase.

Further, a step of drying the nanoparticles by fluidized bed drying may be included. The fluidized bed drying step may comprise heating the nanoparticles. The heating temperature may be about 30-80 °C, or about 30-60, 30-40, 40-80, 60-80 or 40-60 °C, or about 30, 35, 40, 45, 50, 55, 60, 65, 70, 75 or 80°C. The drying step may comprise passing gas flow over the nanoparticles. The gas may be one not reacting with the nanoparticles. For example, it may be air, nitrogen, argon, helium, carbon dioxide or a mixture thereof, and may be dried. The drying step may comprise applying partial vacuum to the nanoparticles. The partial vacuum may be an absolute pressure of about 0.01-0.5 atm, or about 0.01-0.1, 0.01-0.05, 0.1-0.5, 0.25 -0.5, 0.05-0.1 or 0.1-0.25 atm, or about 0.01, 0.05, 0.1, 0.2, 0.3, 0.4 or 0.5 atm.

In an embodiment of the present invention, stearic acid and soy lecithin are dissolved after removing alcohol groups to prepare an aqueous phase (W/O type), which is added to an oil phase (O/W type) to obtain a modified starch suspension. Then, the suspension is dispersed and passed through a filter to prepare nanoparticles with size ranging from 100 to 200 nm (see Example 1-2).

Such prepared nanoparticles are coated on the surface of the double-coated lactic acid bacteria of the step (c), thereby improving viability of the lactic acid bacteria. Preferably, the nanoparticles may be used in an amount of 0.1-10 weight%, more preferably 0.5-3 weight%, based on the total weight of the double-coated lactic acid bacteria of the step (c).

For the triple coating such prepared nanoparticles, a commonly used nanoparticle coating method can be used. Those skilled in the art may easily select the coating method depending on the particular nanoparticles and lactic acid bacteria. The commonly used nanoparticle coating method may be any one as long as the activity of the lactic acid bacteria is maintained. For example, although not limited thereto, physical coating methods (gas evaporation-condensation, mechanical synthesis, application) or chemical coating methods (precipitation, spray drying, solid-phase synthesis) may be used. Physical coating is preferred. The physical coating may comprise, although not limited thereto, applying the nanoparticles on the surface of lactic acid bacteria.

In an embodiment of the present invention, triple-coated lactic acid bacteria are prepared from the double-coated lactic acid bacteria of the step (c) and the nanoparticles using a circulating fluidized bed dry coater (CFBDC) (see Example 1-2).

In the step (d), the surface of the lactic acid bacteria is completely coated by the nanoparticles (see Test Example 1-4). The resultant triple-coated lactic acid bacteria have outstandingly better acid resistance (see Test Example 1-1), bile resistance (see Test Example 1-2) and stability in accelerated test (see Test Example 1-3) than conventional double-coated lactic acid bacteria. In addition, they exhibit significantly better viability when stored at 4°C, 25 °C or 37 °C (see Test Example 1-5 and Test Example 1-6), or upon sterilization (see Test Example 1-7) than conventional double-coated lactic acid bacteria.

The present invention further provides triple-coated lactic acid bacteria prepared by the aforesaid method. Therefore, the triple-coated lactic acid bacteria according to the present invention have outstandingly better acid resistance, bile resistance, stability in accelerated test, and so forth, as compared to conventional non-coated, single-coated or double-coated lactic acid bacteria, and can maintain their physiological activities without being destroyed by gastric acid or bile acid.

The present invention further provides an article comprising the triple-coated lactic acid bacteria. Although not limited thereto, the article may be selected from the group consisting of fermented milk, processed milk, fermented soybean products, fermented kimchi products, functional drinks, functional foods, conventional foods, medicines and cosmetics.

### [Mode for the Invention]

Hereinafter, the present invention is described in further detail through examples. However, the following examples are provided for illustrative purposes only, and they do not limit the present invention.

### Example 1: Preparation of inventive triple-coated lactic acid bacteria coated with nanoparticles

### 1-1. Preparation of conventional double-coated lactic acid bacteria coated with protein and polysaccharide

First, lactic acid bacteria *Lactobacillus acidophilus* (CBT-LH) single-coated with protein were prepared as follows.

4 kg of skim milk powder and 2 kg of soy protein isolate were suspended in 100 kg of water. In an enzyme treatment tank equipped with a low speed agitator and a temperature and pH controller, 1.02 g of a protease (Protease-N, Amano) dissolved in 100 mL water was added at 55°C (initial pH 7.0). Hydrolysis was carried out until pH reached 6.0. Following the hydrolysis, 5 kg of glucose, 1 kg of meat extract, 0.5 kg of yeast extract, 50 g of dipotassium phosphate, 50 g of ammonium citrate, 50 g of sodium acetate, 10 g of magnesium sulfate and 10 g of manganese sulfate were added and dissolved. The solution was transferred to a 200 L anaerobic fermentation tube, sterilized at 121°C for 15 minutes, and then inoculated with 2 L of an inoculum. The resulting solution was fermented for 12 hours while maintaining pH at 6.0 using ammonia. The fermented solution was transferred to a continuous centrifuge at a flow rate of 60 L/hr. The lactic acid bacteria and protein remnants were deposited and collected to obtain the lactic acid bacteria *Lactobacillus acidophilus* (CBT-LH) single-coated with protein.

Then, lactic acid bacteria *Lactobacillus acidophilus* (CBT-LH) double-coated with polysaccharide were prepared as follows.

10 L of an aqueous cryoprotectant solution comprising 1 kg of trehalose, 1 kg of mannitol and 1 kg of maltodextrin was prepared by sterilizing under pressure, and 3 L of an aqueous polysaccharide solution in which 10 g of xanthan gum, 10 g of cellulose and 10 g of levan were dissolved was prepared by sterilizing under pressure. After mixing the lactic acid bacteria single-coated with protein, the aqueous cryoprotectant solution and the aqueous polysaccharide solution, the mixed solution was homogenized at 5,000 rpm, and rapidly frozen in a freezer at -55 °C, lyophilized at a temperature ranging from 0°C to 40 °C.

Protein remnants which are semisoluble in water surrounded the lactic acid bacteria, and water-soluble peptide ingredients were deposited on the cell walls of the lactic acid bacteria, thereby completing a protein coating. Polysaccharide ingredients were formed into clusters having a highly compact structure due to their power adhesion to the lactic acid bacteria. As a result, double-coated lactic acid bacteria coated with protein and polysaccharide were prepared.

### 1-2. Preparation of inventive triple-coated lactic acid bacteria coated with nanoparticles

Triple-coated lactic acid bacteria were prepared by coating the double-coated lactic acid bacteria coated with protein and polysaccharide prepared in Example 1-1 using nanoparticles.

First, for the preparation of nanoparticles, stearic acid and soy lecithin were added to a water tank at 70°C in order to remove alcohol groups, and dissolved to prepare an aqueous phase (W/O type). The aqueous phase was added to an oil phase (O/W type) at high temperature of about 80°C using a syringe needle, and mixed at 70-80 °C for 2 hours. The resultant modified starch suspension was cooled to room temperature while resuspending for 300 seconds using an ultrasonic homogenizer, and passed through a Millipore filter to screen out nanoparticles with size ranging from 100 to 200 nm.

Such prepared nanoparticles (2 weight% based on the lactic acid bacteria) and the double-coated lactic acid bacteria prepared in Example 1-1 were passed through a circulating fluidized bed dry coater (FBC-120, ChungJin Tech, Korea) at 30-35 °C for 3-4 hours to prepare the inventive triple-coated lactic acid bacteria coated with nanoparticles.

### Test Example 1: Comparison of inventive triple-coated lactic acid bacteria coated with nanoparticles and conventional double-coated lactic acid bacteria

### 1-1. Comparison of acid resistance

Acid resistance test was carried out for the inventive triple-coated lactic acid bacteria coated with nanoparticles prepared in Example 1-2 and the conventional double-coated lactic acid bacteria prepared in Example 1-1. The result is given in the following Table 1.

Specifically, 3.4x10¹¹ CFU/g of the lactic acid bacteria prepared in Example 1-2 or the lactic acid bacteria prepared in Example 1-1 were treated with artificial gastric juice (pH 2.1) for 30, 60, 90 and 120 minutes. After gradually diluting with physiological saline and smearing on an agar plate, number of lactic acid bacteria was counted.

**[Table 1]**

| Comparison of acid resistance of inventive triple-coated lactic acid bacteria coated with nanoparticles and conventional double-coated lactic acid bacteria | | |
|---|---|---|
| Exposure time (min) | Lactic acid bacteria of Example 1-1 (CFU/g) | Lactic acid bacteria of Example 1-2 (CFU/g) |
| 0 | 3.4x10¹¹ | 3.47x10¹¹ |
| 30 | 3.2x10¹¹ | 3.21x10¹¹ |
| 60 | 3.1x10¹¹ | 3.15x10¹¹ |
| 90 | 3.1x10¹¹ | 3.11x10¹¹ |
| 120 | 2.9x10¹¹ | 3.05x10¹¹ |
| Viability at 120 min (%) | 85.3 | 87.9 |

### 1-2. Comparison of bile resistance

Bile resistance test was carried out for the inventive triple-coated lactic acid bacteria coated with nanoparticles prepared in Example 1-2 and the conventional double-coated lactic acid bacteria prepared in Example 1-1. The result is given in the following Table 2.

Specifically, 3.5x10¹¹ CFU/g of the lactic acid bacteria prepared in Example 1-2 or the lactic acid bacteria prepared in Example 1-1 were treated with 0.5% oxgall solution for 30, 60, 90 and 120 minutes. After gradually diluting with physiological saline and smearing on an agar plate, number of lactic acid bacteria was counted.

**[Table 2]**

| Comparison of bile resistance of inventive triple-coated lactic acid bacteria coated with nanoparticles and conventional double-coated lactic acid bacteria | | |
|---|---|---|
| Exposure time (min) | Lactic acid bacteria of Example 1-1 (CFU/g) | Lactic acid bacteria of Example 1-2 (CFU/g) |
| 0 | 3.5x10¹¹ | 3.60x10¹¹ |
| 30 | 3.3x10¹¹ | 3.40x10¹¹ |
| 60 | 3.2x10¹¹ | 3.35x10¹¹ |
| 90 | 3.0x10¹¹ | 3.20x10¹¹ |
| 120 | 2.9x10¹¹ | 3.15x10¹¹ |
| Viability at 120 min (%) | 82.9 | 87.5 |

### 1-3. Comparison of stability in accelerated test

Accelerated test was carried out for the inventive triple-coated lactic acid bacteria coated with nanoparticles prepared in Example 1-2 and the conventional double-coated lactic acid bacteria prepared in Example 1-1. The result is given in the following Table 3.

Specifically, 3.1x10¹¹ CFU/g of the lactic acid bacteria prepared in Example 1-2 or the lactic acid bacteria prepared in Example 1-1 were kept under the condition of 40 °C and relative humidity 70% for 10, 20, 30 and 40 days. After gradually diluting with physiological saline and smearing on an agar plate, number of lactic acid bacteria was counted.

**[Table 3]**

| Comparison of stability in accelerated test of inventive triple-coated lactic acid bacteria coated with nanoparticles and conventional double-coated lactic acid bacteria | | |
|---|---|---|
| Time (days) | Lactic acid bacteria of Example 1-1 (CFU/g) | Lactic acid bacteria of Example 1-2 (CFU/g) |
| 0 | 3.1x10¹¹ | 3.22x10¹¹ |
| 10 | 2.9x10¹¹ | 2.95x10¹¹ |
| 20 | 2.6x10¹¹ | 2.80x10¹¹ |
| 30 | 2.5x10¹¹ | 2.65x10¹¹ |
| 40 | 2.0x10¹¹ | 2.37x10¹¹ |
| Viability at day 40 (%) | 64.5 | 73.6 |

### 1-4. Comparison of shape

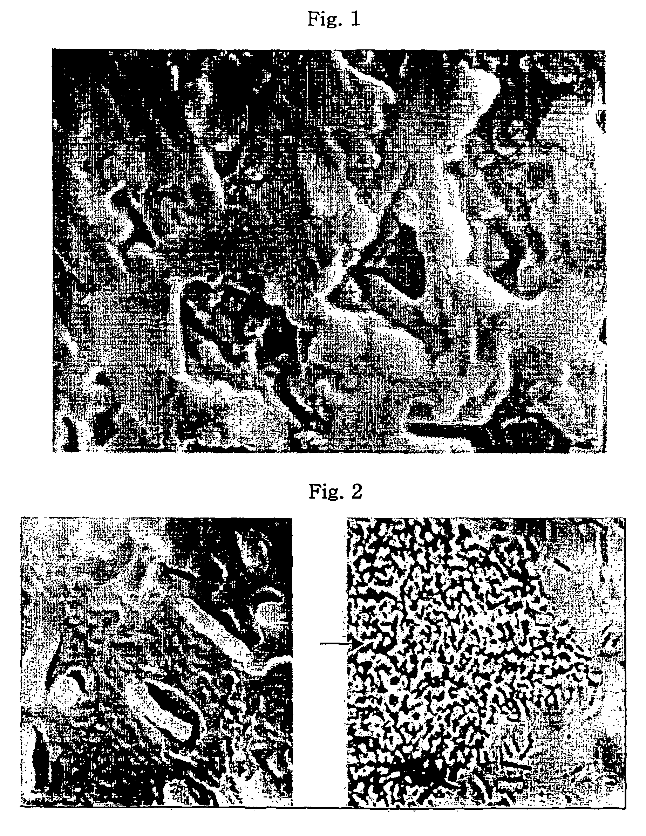

Shapes of the inventive triple-coated lactic acid bacteria coated with nanoparticles prepared in Example 1-2 and the conventional double-coated lactic acid bacteria were compared (Fig. 1 and Fig. 2).

As seen in Fig. 1 and Fig. 2, the lactic acid bacteria double-coated by the conventional method were less compact coating and a relatively large portion of the lactic acid bacteria was exposed. In contrast, the lactic acid bacteria coated with nanoparticles according to the present invention exhibited compact coating and a relatively small portion of the lactic acid bacteria was exposed.

### 1-5. Storage at 4 °C

After storing the inventive triple-coated lactic acid bacteria coated with nanoparticles prepared in Example 1-2 and the conventional double-coated lactic acid bacteria prepared in Example 1-1 at 4 °C for 1 year, number of lactic acid bacteria per 1 mL depending on time was counted. The result is given in the following Table 4.

**[Table 4]**

| Storage of inventive triple-coated lactic acid bacteria coated with nanoparticles and conventional double-coated lactic acid bacteria at 4 °C | | |
|---|---|---|
| Time (days) | Lactic acid bacteria of Example 1-1 (CFU/mL) | Lactic acid bacteria of Example 1-2 (CFU/mL) |
| 0 | 1.95x10¹¹ | 2.15x10¹¹ |
| 45 | 1.53x10¹¹ | 1.99x10¹¹ |
| 90 | 1.29x10¹¹ | 1.95x10¹¹ |
| 180 | 1.10x10¹¹ | 1.88x10¹¹ |
| 365 | 9.70x10¹⁰ | 1.89x10¹¹ |
| Viability at day 365 (%) | 49.7 | 87.9 |

### 1-6. Storage at 25°C and 37 °C

After storing the inventive triple-coated lactic acid bacteria coated with nanoparticles prepared in Example 1-2 and the conventional double-coated lactic acid bacteria prepared in Example 1-1 at 25°C for 1 year, number of lactic acid bacteria per 1 mL was counted. The result is given in the following Table 5.

**[Table 5]**

| Storage of inventive triple-coated lactic acid bacteria coated with nanoparticles and conventional double-coated lactic acid bacteria at 25°C | | |
|---|---|---|
| Time (days) | Lactic acid bacteria of Example 1-1 (CFU/mL) | Lactic acid bacteria of Example 1-2 (CFU/mL) |
| 0 | 1.95x10¹¹ | 2.15x10¹¹ |
| 45 | 1.16x10¹¹ | 1.75x10¹¹ |
| 90 | 8.32x10¹⁰ | 1.58x10¹¹ |
| 180 | 6.83x10¹⁰ | 1.35x10¹¹ |
| 365 | 2.94x10¹⁰ | 1.24x10¹¹ |
| Viability at day 365 (%) | 15.8 | 57.7 |

### [Industrial Applicability]

The lactic acid bacteria prepared in accordance with the present invention are not killed by gastric acid or bile acid upon uptake, and maintain their inherent physiological activities. Therefore, they can be effectively used in various products, including fermented milk, processed milk, fermented soybean products, fermented kimchi products, functional drinks, functional foods, conventional foods, cosmetics, and the like.

Although the preferred embodiments of the present invention have been disclosed for illustrative purposes, those skilled in the art will appreciate that various modifications, additions and substitutions are possible, without departing from the invention as disclosed in the accompanying claims.

## Claims

1. (Amended) A method for preparing triple-coated lactic acid bacteria comprising the steps of:
(a) treating an aqueous protein solution with a protease;
(b) adding glucose, yeast extract, meat extract, an ionic component and lactic acid bacteria to the aqueous protein solution enzyme-treated in the step (a), and fermenting the solution to prepare single-coated lactic acid bacteria;
(c) mixing the single-coated lactic acid bacteria prepared in the step (b) with an aqueous polysaccharide solution to prepare double -coated lactic acid bacteria; and
(d) coating the double-coated lactic acid bacteria prepared in the step (c) with nanoparticles to prepare triple-coated lactic acid bacteria, wherein said nanoparticles are solid lipid nanoparticles.

2. The method for preparing triple-coated lactic acid bacteria as set forth in claim 1, wherein the aqueous protein solution is 1-10% aqueous solution prepared from soy protein isolate or skimmed milk powder.

3. The method for preparing triple-coated lactic acid bacteria as set forth in claim 1, wherein the glucose, the yeast extract, the meat extract, the ionic component and the lactic acid bacteria of step (b) is added in an amount of 1-5 weight%, 0.1-1.5 weight%, 0.1-1.5 weight%, and 0.01-0.1 weight%, based on the weight of the aqueous protein solution enzyme-treated in the step (a), respectively.

4. The method for preparing triple-coated lactic acid bacteria as set forth in claim 1, wherein the ionic component of the step (b) is at least one selected from the group consisting of ammonium citrate, sodium acetate, dipotassium phosphate, magnesium sulfate, manganese sulfate and sodium chloride.

5. The method for preparing triple-coated lactic acid bacteria as set forth in claim 1, wherein the lactic acid bacteria of the step (b) are at least one selected from the group consisting of genus *Streptococcus,* genus *Lactococcus,* genus *Enterococcus,* genus *Lactobacillus,* genus *Pediococcus*, genus *Leuconostoc,* genus *Weissella* and genus *Bifidobacterium.*

6. The method for preparing triple-coated lactic acid bacteria as set forth in claim 1, wherein the lactic acid bacteria of the step (b) are concentrated to a concentration of 10¹⁰-10¹¹ CFU/mL.

7. The method for preparing triple-coated lactic acid bacteria as set forth in claim 1, wherein the aqueous polysaccharide solution of the step (c) is an aqueous polysaccharide solution comprising 1-10% of polysaccharide based on the weight of the single-coated lactic acid bacteria of the step (b).

8. The method for preparing triple-coated lactic acid bacteria as set forth in claim 1, wherein the polysaccharide of the step (c) is at least one polysaccharide selected from the group consisting of xanthan gum, cellulose and levan.

9. The method for preparing triple-coated lactic acid bacteria as set forth in claim 1, which further comprises a step of adding an aqueous cryoprotectant solution to the double-coated lactic acid bacteria of the step (c) and then carrying out lyophilization.

10. The method for preparing triple-coated lactic acid bacteria as set forth in claim 9, wherein the aqueous cryoprotectant solution is an aqueous solution comprising 1-10% of a cryoprotectant based on the weight of the single-coated lactic acid bacteria of the step (b).

11. The method for preparing triple-coated lactic acid baceria as set forth in claim 1, wherein the solid lipid nanoparticles are at least one selected from the group consisting of lecithin, cholesterol, stearic acid, calcium stearate and a combination thereof.

12. The method for preparing triple-coated lactic acid bacteria as set forth in claim 1, wherein the nanoparticles of the step (d) are added in an amount of 0.5-3 weight% based on the weight of the double-coated lactic acid bacteria of the step (c).

13. Triple-coated lactic acid bacteria prepared by the method as set forth in any of claims 1 to 12.

14. An article comprising the triple-coated lactic acid bacteria as set forth in claim 13.

15. The article as set forth in claim 14, which is selected from fermented milk, processed milk, fermented soybean products, fermented kimchi products, functional drinks, functional foods, conventional foods, medicines and cosmetics.

## Patentansprüche

1. Verfahren zur Herstellung von dreifach ummantelten Milchsäurebakterien umfassend die Schritte von:
a) Behandeln einer wasserhaltigen Proteinlösung mit einer Protease;
b) Hinzufügen von Glukose, Hefeextrakt, Fleischextrakt, einer ionischen Komponente und Milchsäurebakterien zu der, unter Schritt a) Enzym behandelten, wasserhaltigen Proteinlösung, und Fermentierung der Lösung, um einfachummantelte Milchsäurebakterien herzustellen;
c) Mischen der einfach ummantelten Milchsäurebakterien, die in dem Schritt b) hergestellt wurden, mit einer wasserhaltigen Polysaccharidlösung, um zweifach ummantelte Milchsäurebakterien herzustellen; und
d) Ummanteln der zweifach ummantelten Milchsäurebakterien, die in dem Schritt c) hergestellt wurden, mit Nanopartikeln, um dreifach ummantelte Milchsäurebakterien herzustellen, wobei die Nanopartikel Fest-Lipid-Nanopartikel sind.

2. Verfahren zur Herstellung von dreifach ummantelten Milchsäurebakterien gemäß Anspruch 1,
**dadurch gekennzeichnet, dass**
die wasserhaltige Proteinlösung 1-10 % wasserhaltige Lösung hergestellt aus Sojaproteinisolat oder entrahmten Milchpulver ist.

3. Verfahren zur Herstellung von dreifach ummantelten Milchsäurebakterien gemäß Anspruch 1,
**dadurch gekennzeichnet, dass**
die Glukose, das Hefeextrakt, das Fleischextrakt, die ionische Komponente und die Milchsäurebakterien gemäß Schritt b) in einer Menge von 1-5 Gewichtsprozent, 0,1-1,5 Gewichtsprozent, 0,1-1,5 Gewichtsprozent und 0,01-0,1 Gewichtsprozent, basierend auf dem Gewicht der enzymbehandelten wasserhaltigen Proteinlösung gemäß Schritt a), entsprechend hinzugefügt wird.

4. Verfahren zur Herstellung von dreifach ummantelten Milchsäurebakterien gemäß Anspruch 1,
**dadurch gekennzeichnet, dass**
die ionische Komponente von Schritt b) zumindest eine ausgewählt aus der Gruppe bestehend aus Ammoniumcitrat, Natriumacetat, Di-Kaliumphosphat, Magnesiumsulfat, Mangansulfat und Natriumchlorid ist.

5. Verfahren zur Herstellung von dreifach ummantelten Milchsäurebakterien gemäß Anspruch 1,
**dadurch gekennzeichnet, dass**
die Milchsäurebakterien des Schrittes b) zumindest eines aus der Gruppe bestehend aus der Gattung *Streptococcus, Lactococcus, Enterococcus, Lactobacillus, Pediococcus, Leuconostoc, Weissella* und *Bifidobacterium* sind.

6. Verfahren zur Herstellung von dreifach ummantelten Milchsäurebakterien gemäß Anspruch 1,
**dadurch gekennzeichnet, dass**
die Milchsäurebakterien des Schrittes b) zu einer Konzentration von 10¹⁰-10¹¹ CFU/mL konzentriert sind.

7. Verfahren zur Herstellung von dreifach ummantelten Milchsäurebakterien gemäß Anspruch 1,
**dadurch gekennzeichnet, dass**
die wasserhaltige Polysaccharidlösung von Schritt c) eine wasserhaltige Polysaccharidlösung umfassend 1-10 % Polysaccharid basierend auf dem Gewicht der einfach ummantelten Milchsäurebakterien von Schritt b) ist.

8. Verfahren zur Herstellung von dreifach ummantelten Milchsäurebakterien gemäß Anspruch 1,
**dadurch gekennzeichnet, dass**
die Polysaccharide des Schritts c) zumindest ein Polysaccharid ausgewählt aus der Gruppe bestehend aus Xanthan-Gummi, Zellulose und Lävan ist.

9. Verfahren zur Herstellung von dreifach ummantelten Milchsäurebakterien gemäß Anspruch 1,
**dadurch gekennzeichnet, dass**
es weiterhin einen Schritt des Hinzufügens einer wasserhaltigen Gefrierschutzmittellösung zu den zweifach ummantelten Milchsäurebakterien des Schrittes c) und dann Durchführung von Lyophilisation umfasst.

10. Verfahren zur Herstellung von dreifach ummantelten Milchsäurebakterien gemäß Anspruch 9,
**dadurch gekennzeichnet, dass**
die wasserhaltige Gefrierschutzmittellösung eine wasserhaltige Lösung umfassend 1-10 % eines Gefrierschutzmittels basierend auf dem Gewicht der einfach ummantelten Milchsäurebakterien gemäß Schritt b) ist.

11. Verfahren zur Herstellung von dreifach ummantelten Milchsäurebakterien gemäß Anspruch 1,
**dadurch gekennzeichnet, dass**
die Fest-Lipiden-Nanopartikel zumindest eines ausgewählt aus der Gruppe bestehend aus Lecithin, Cholesterol, Stearinsäure, Calciumstearat und einer Kombination davon sind.

12. Verfahren zur Herstellung von dreifach ummantelten Milchsäurebakterien gemäß Anspruch 1,
**dadurch gekennzeichnet, dass**
die Nanopartikel nach Schritt d) in einer Menge von 0,5-3 Gewichtsprozent basierend auf dem Gewicht der zweifach ummantelten Milchsäurebakterien des Schrittes c) hinzugefügt werden.

13. Dreifach ummantelte Milchsäurebakterien hergestellt durch das Verfahren gemäß einem der Ansprüche 1 bis 12.

14. Erzeugnis umfassend die dreifach ummantelten Milchsäurebakterien gemäß Anspruch 13.

15. Erzeugnis gemäß Anspruch 14, welches ausgewählt ist aus fermentierter Milch, verarbeiteter Milch, fermentierter Sojabohnenprodukte, fermentierter Kimchiprodukte, funktionaler Getränke, funktionaler Nahrungsmittel, konventioneller Nahrungsmittel, Medizin und Kosmetika.

## Revendications

1. Procédé de préparation de bactéries d'acide lactique triplement revêtues comprenant les étapes de :
(a) traitement d'une solution aqueuse de protéine avec une protéase ;
(b) ajout de glucose, d'extrait de levure, d'extrait de viande, d'un composant ionique et de bactéries d'acide lactique à la solution aqueuse de protéine traitée par enzyme à l'étape (a), et fermentation de la solution afin de préparer des bactéries d'acide lactique à un seul revêtement ;
(c) mélange des bactéries d'acide lactique à un seul revêtement préparées à l'étape (b) avec une solution aqueuse de polysaccharide afin de préparer des bactéries d'acide lactique à double revêtement ; et
(d) revêtement des bactéries d'acide lactique à double revêtement préparées à l'étape (c) avec des nanoparticules afin de préparer des bactéries d'acide lactique à triple revêtement, où lesdites nanoparticules sont des nanoparticules de lipide solide.

2. Procédé de préparation de bactéries d'acide lactique triplement revêtues selon la revendication 1, dans lequel la solution aqueuse de protéine est une solution aqueuse à 1 à 10 % préparée à partir d'isolat de protéine de soja ou de poudre de lait écrémé.

3. Procédé de préparation de bactéries d'acide lactique triplement revêtues selon la revendication 1, dans lequel le glucose, l'extrait de levure, l'extrait de viande, le composant ionique et les bactéries d'acide lactique de l'étape (b) sont ajoutés dans une quantité de 1 à 5 % en poids, 0,1 à 1,5 % en poids, 0,1 à 1,5 % en poids, et 0,01 à 0,1 % en poids, sur la base du poids de la solution aqueuse de protéine traitée par enzyme à l'étape (a), respectivement.

4. Procédé de préparation de bactéries d'acide lactique triplement revêtues selon la revendication 1, dans lequel le composant ionique de l'étape (b) est au moins l'un choisi dans le groupe consistant en le citrate d'ammonium, l'acétate de sodium, le phosphate dipotassique, le sulfate de magnésium, le sulfate de manganèse et le chlorure de sodium.

5. Procédé de préparation de bactéries d'acide lactique triplement revêtues selon la revendication 1, dans lequel les bactéries d'acide lactique de l'étape (b) sont au moins l'un choisi dans le groupe consistant en le genre *Streptococcus,* le genre *Lactococcus,* le genre *Enterococcus,* le genre *Lactobacillus,* le genre *Pediococcus,* le genre *Leuconostoc,* le genre *Weissella* et le genre *Bifidobacterium.*

6. Procédé de préparation de bactéries d'acide lactique triplement revêtues selon la revendication 1, dans lequel les bactéries d'acide lactique de l'étape (b) sont concentrées à une concentration de 10¹⁰ - 10¹¹ CFU/mL.

7. Procédé de préparation de bactéries d'acide lactique triplement revêtues selon la revendication 1, dans lequel la solution aqueuse de polysaccharide de l'étape (c) est une solution aqueuse de polysaccharide comprenant 1 à 10 % de polysaccharide sur la base du poids des bactéries d'acide lactique simplement revêtues de l'étape (b).

8. Procédé de préparation de bactéries d'acide lactique triplement revêtues selon la revendication 1, dans lequel le polysaccharide de l'étape (c) est au moins un polysaccharide choisi dans le groupe consistant en la gomme de xanthane, la cellulose et le lévane.

9. Procédé de préparation de bactéries d'acide lactique triplement revêtues selon la revendication 1, qui comprend en outre une étape d'ajout d'une solution aqueuse de cryoprotecteur aux bactéries d'acide lactique doublement revêtues de l'étape (c), puis la réalisation d'une lyophilisation.

10. Procédé de préparation de bactéries d'acide lactique triplement revêtues selon la revendication 9, dans lequel la solution aqueuse de cryoprotecteur est une solution aqueuse comprenant 1 à 10 % d'un cryoprotecteur sur la base du poids des bactéries d'acide lactique simplement revêtues de l'étape (b).

11. Procédé de préparation de bactéries d'acide lactique triplement revêtues selon la revendication 1, dans lequel les nanoparticules de lipide solide sont au moins l'un choisi dans le groupe consistant en la lécithine, le cholestérol, l'acide stéarique, le stéarate de calcium et une combinaison de ceux-ci.

12. Procédé de préparation de bactéries d'acide lactique triplement revêtues selon la revendication 1, dans lequel les nanoparticules de l'étape (d) sont ajoutées dans une quantité de 0,5 à 3 % en poids sur la base du poids des bactéries d'acide lactique doublement revêtues de l'étape (c).

13. Bactéries d'acide lactique triplement revêtues préparées par le procédé tel qu'énoncé dans l'une quelconque des revendications 1 à 12.

14. Article comprenant les bactéries d'acide lactique triplement revêtues telles qu'énoncées à la revendication 13.

15. Article selon la revendication 14, qui est choisi parmi le lait fermenté, le lait transformé, les produits de soja fermenté, les produits de kimchi fermenté, les boissons fonctionnelles, les aliments fonctionnels, les aliments classiques, les remèdes et les cosmétiques.
